Europäisches Patentamt

European Patent Office

Office européen des brevets

(11) Publication number: **0 068 554**
**B1**

(12) **EUROPEAN PATENT SPECIFICATION**

(45) Date of publication of patent specification: **13.11.85**

(21) Application number: **82200732.4**

(22) Date of filing: **14.06.82**

(51) Int. Cl.⁴: **B 01 J 21/16,** B 01 J 21/12, C 07 C 2/20, B 01 J 27/16

(54) **Heterogeneous cationic catalytic systems suitable for the oligomerisation of linear non-terminal olefins, and a process for preparing said oligomers using said systems.**

(30) Priority: **18.06.81 IT 2239881**

(43) Date of publication of application:
**05.01.83 Bulletin 83/01**

(45) Publication of the grant of the patent:
**13.11.85 Bulletin 85/46**

(84) Designated Contracting States:
**AT BE CH DE FR GB LI LU NL SE**

(56) References cited:
**DE-C- 947 967**

(73) Proprietor: **ANIC S.p.A.**
**Via Ruggero Settimo, 55**
**I-90139 Palermo (IT)**

(72) Inventor: **Priola, Aldo**
**Via M. di Cefalonia 40**
**I-20097 S. Donato Milanese (Milan) (IT)**
Inventor: **Fattore, Vittorio**
**Via Kennedy 27**
**I-20097 S. Donato Milanese (Milan) (IT)**
Inventor: **Arrighetti, Sergio**
**Via Trieste 16**
**I-20097 S. Donato Milanese (IT)**
Inventor: **Mancini, Giuseppe**
**Via degli Olmi 1**
**I-20077 Melegnano (Milan) (IT)**

(74) Representative: **Roggero, Sergio et al**
**Ing. Barzanò & Zanardo Milano S.p.A. Via**
**Borgonuovo 10**
**I-20121 Milano (IT)**

Courier Press, Leamington Spa, England.

## Description

This invention relates to an improved process for preparing non-terminal olefin oligomers.

It is known, from DE—C—947 967 that a composition of a $BF_3$-inorganic acid complex and gaseous $BF_3$ is suitable for the oligomerization of olefins, and it is also known that such a complex is readily formed by contacting inorganic acids with gaseous $BF_3$.

The present Applicants have now discovered that it is possible to catalytically oligomerize non-terminal olefins with better results without preforming any $BF_3$-acid complex, contrary to said prior art teachings.

According to the invention, a process for the oligomerization of non-terminal C10—C20 olefins is suggested, in the presence of phosphoric acid and boron trifluoride, characterized in that the catalysts used is a substrate of silica, alumina or active carbon having a specific surface area over 200 $m^2/g$ and an overall pore volume over 0,5 ml/g impregnated with phosphoric acid ($H_3PO_4$) in a quantity, expressed as $P_2O_5$ of from 20 to 100 parts by weight per 100 parts by weight of the substrate, the reaction taking place in the liquid phase in the presence of gaseous $BF_3$ under a pressure of from 0,3 to 10 bar at a temperature of from 10°C to 130°C, optionally in the presence of a solvent.

According to the prior art, starting from 1-decene, a product is obtained, which mainly consists of a C-30 trimer, which, upon hydrogenation, is an excellent base for synthetic lubricating oils having optimum properties as to viscosity index, kinematic viscosity at 210°F (99°C), volatility, pour point and other desirable characteristics. Such products have thus been very favourably received in the synthetic lubricant field, and their use finds an evergrowingly wide acceptance in the market.

The exceptional rheological properties of such products are due both to the presence of branches of an appropriate length in their molecules and to the stringent control of their molecular weight so as to prevent both the presence of substances having a low molecular weight, which would render the end product too volatile, and the presence of higher oligomers, which would render the final product too tacky.

Alpha-olefin oligomers, however, are costly because of the high first cost of the starting alpha-olefins, so that the felt is wanted of products having favourable rheological properties while being cheaper and susceptible of being made from more readily available oligomers.

By adopting the Applicant's procedure as outlined above, it has become possible to prepare products having properties similar to those of the products previously prepared from 1-decene as the starting material, but starting, instead, from appropriate types of linear non-terminal olefins: Applicants use a particular cationic catalyst system which has been defined in the foregoing.

Therefore, products are obtained, which, upon hydrogenation, have outstanding properties as lubricant bases for synthetic lubricants while starting from raw materials which are cheaper than the alpha-olefins as used hitherto, and which can be obtained from linear-alkane dehydrogenation processes, such as the well known Olex process. It is also known that non-terminal olefins can be oligomerized with Friedel-Crafts catalysts, but the product thus obtained exhibit widely scattered molecular weigh values, it being likewise known that it is difficult, with such conventional methods, to obtain lubricants of an acceptable quality without resorting to expensive fractionation runs which, inter alia, originate large amounts of undesirable by-products.

The present Applicants are also proprietors of an Italian Patent Application No 20106 A/80, filed on February 22, 1980, made available to the public on 22. Aug. 1981, relating to the selective oligomerization of non-terminal olefins, so that only dimers and/or trimers are obtained. Therefore, starting from suitable non-terminal olefin cuts, products are obtained which contain but small amounts of unusable fractions.

The advantages which can be obtained in this way are:

1) Dispensing with fractionation runs;

2) Thorough exploitation of the non-terminal olefin monomers, and

3) Obtaining oligomers having excellent rheological properties, which are definitely superior to those obtainable with the conventional Friedel-Crafts procedures.

In the particular applications contemplated herein, the value of the kinematic viscosity at 100°C is very important as it must lie within a comparatively narrow range, that is, from 3 to 6 centistokes, corresponding to $3.10^{-6}$ to $6.10^{-6}$ $m^2/s$, for the oil being susceptible of profitable use as a lubricant base for synthetic lubricants. Moreover, the Viscosity Index must be high, the pour point low and the open-cup flammability point must be above 200°C.

The process disclosed in said Italian Patent Application uses a catalyst system composed of adducts of $AlCl_3$ with esters, complexes of $BF_3$ with alcohols or acids (both organic and inorganic), or also dispersions of $AlCl_3$ on specially selected substrates.

In spite of doubtless advantages, however, the following drawbacks have been experienced with the aforementioned approach, namely:

1) The catalysts based on adducts of $AlCl_3$ with esters interact with the monomers and form a viscous liquid which is insoluble in the reaction medium, so that oligomerization is hampered and the possibility of reusing the catalyst becomes very poor. Moreover, a high temperature, above 140°C, is required and the dimer selectivity of the reaction is poor.

2) The catalyst based on $BF_3$-complexes with alcohols or acids form likewise a viscous liquid, and the difficulties are of the same kind as outlined above, and, finally,

3) The dispersions of AlCl₃ on silica or alumina require a high temperature, above 120°C, and the dimer selectivity grows poor again.

By using, instead, the process according to the present invention, one obtains the following outstanding advantages:

1) The solid catalyst remains finely dispersed on the surface of a substrate during progress of the reaction, so that oligomerization proceeds by solid-liquid interaction upon the contact of the catalyst with the monomer: therefore, the catalyst can readily be separated from the reaction mixture once the reaction is completed and such an operation is both easy and convenient. The reaction velocity is higher and the reaction trend is both smooth and even.

2) The catalyst can be reused in subsequent oligomerization runs, if so desired, provided that the used up $BF_3$ is replaced, so that the catalyst consumption can be reduced considerably.

· 3) The removal of the sludge, or pitch, if any is collected on the reactor's bottom, becomes easier.

4) The overall process is simplified and this is an asset especially when continuous operation is adopted.

5) The selectivity provided by the catalyst system in question is so high that the final product can be used, upon subsequent hydrogenation, as such in the formulation of engine lubricants without requiring any distillation step, it being merely necessary to remove the unreacted monomer and small amounts of volatile matters.

Non-terminal olefins which may be used in the process of this invention comprise linear monoolefins from C10 to C20, containing an unsaturated olefin bond anywhere in their molecules, and the configuration of the unsaturated olefin bond can indifferently be of the cis- or the trans-type. The commercially available cuts are, generally C11—C14 mixtures, C15—C18 mixtures and the like.

The oligomerization reaction according to the invention can optionally be carried out in a solvent medium; preferred are linear or branched saturated aliphatic hydrocarbons such as n-pentane, n-hexane, n-heptane and mixtures thereof. The reaction temperature is from 10°C to 130°C, and the reaction time may be from 1 to 8 hours. The gas-chromatographic analysis, coupled with mass-spectrometry, has shown that the reaction products are mainly dimers, the trimers being present in comparatively small amounts and can be stripped from unreacted monomers by appropriate vacuum-distillation operations. The distillation residue can either be analyzed or can be vacuum-distilled: if so a small fraction, about 5% by weight, of high-boiling products is discarded. For being used in the synthetic lubricant field the oligomers so produced must be hydrogenated, which can be made by any appropriate procedure, and it has been ascertained that hydrogenation does not affect the rheological properties of the oligomers appreciably. For the oligomers produced according to the process of the invention, the physico-chemical and technological properties have been determined as follows.

The degree of unsaturation is expressed in terms of the bromine number (g of Br absorbed by 100 g of oil) and has been determined as described in "Organic Analysis", Vol. III, Interscience Publ., New York (1956). The molecular-weight distribution was determined by Gel-Permeation Chromatography (GPC) using the Waters instrument, Model ALC/GPC-201, in toluene.

The kinematic viscosity was determined both at 100°F (38°C) and 210°F (99°C) with the ASTM D445-74 method. The Viscosity Index (V.I.) was calculated with the ASTM D2270-75 method, and the pour point with the ASTM D97 method. The open-cup flammability temperature was determined by the ASTM D-92 method.

A few examples will better illustrate the practical aspects of the invention.

Example 1

50g of activated carbon small cylinders (4 mm dia.), having a specific superficial area of 1400 m²/g and a pore volume of 0,8 ml/g are oven-dried at 120°C for 4 hours. 65 g of an 85%-$H_3PO_4$ aqueous solution are weighed and slowly poured onto the activated carbon, the latter being stirred continually. The acid is completely absorbed by the pores of the carbon substrate, whereafter the sample is oven-dried at 120°C for 14 hours in air. The acid content, calculated in terms of $P_2O_5$ of the substrate is 80 g per 100 g of substrate and 1,8 g of the catalyst so prepared are withdrawn and subjected to a vacuum of about 27 N/m² residual pressure at 120°C for 2 hours. The catalyst system is now brought back to the atmospheric pressure with dry nitrogen and, still under a dry nitrogen blanket, is transferred into a cylindrical basket-like container consisting of stainless-steel mesh, the mesh openings being ASTM-20 size. The container is suspended within a 200-ml autoclave fitted with a magnetic stirrer, the autoclave being charged with 100 ml of a fraction composed of C13—C16 non-terminal olefins, fed under a dry nitrogen blanket, the composition of the fraction being, on a weight% basis, as follows:

C13=23,7; C14=35,1; C15=26,8; C16=14.4.

The double bonds are predominantly of the trans-type (61%) and are statistically arranged in each position of the molecules. The autoclave is thermostatically controlled at 100°C and gaseous $BF_3$ is fed from a bottle until a final pressure of about 3 bar obtains in the autoclave. A temperature rise by 6°C is observed, and it is reabsorbed by the system within ten minutes or so. The reaction is continued with stirring, while keeping the temperature steadily at 100°C during 5 hours: with the lapse of this time, the $BF_3$ pressure drops to about 0,8 bar. Stirring is discontinued and the reaction product is discharged, whereafter 100 ml of fresh monomer are introduced and the BF₃ pressure is brought back to its initial value. Such a procedure is reiterated and the BF₃ pressure remains virtually constant throughout the reaction, whereupon the procedure is repeated thrice again and the BF₃ pressure is not varied. The products as obtained in the several runs have been quenched with aqueous ammonia and then individually analyzed, whereupon the following results have been obtained:

|  | a) % conversion | b) % selectivity |
|---|---|---|
| 1st run | 80 | 84 |
| 2nd run | 81 | 82 |
| 3rd run | 79 | 79 |
| 4th run | 78 | 79 |
| 5th run | 75 | 78 |

a) Determined by Gas-chromatography(GC); b) dimer versus dimer+higher oligomer: the ratio also determined by GC.

The quantity of $H_3PO_4$ collected on the reactor bottom after the five runs was acidimetrically titrated and was as low as less than 2% of the total amount of $H_3PO_4$ present in the system.

It can be seen that the catalyst performance was virtually unaltered throughout the five runs and that there was no appreciable sign of ageing or poisoning of the catalyst system. The products obtained during the several runs were combined together and washed with 3%-aqueous ammonia, and then with water to neutrality. They were subsequently subjected to a vacuum-treatment at 27 N/m² residual pressure to remove the unreacted monomers (21%), whereafter they were mass-hydrogenated in the presence of carbon-supported Pd (5% of Pd) to the extent of 4% by weight relative to the oil, at a temperature of 150°C and under a hydrogen pressure of about 10 bars for 5 hours. The catalyst can be separated from the hydrogenated oil by filtration and another vacuum-treatment is applied to the oil (160°C, vacuum 27 N/m²) to remove the last traces of volatile matters: these amounted to 2% approx.

The physico-chemical and technological properties of the product have been determined and were found as follows:

d=0,8284 g/ml Br-number=0,31 g per 100 g of oil.
· Flammability point (open cup)=205°C.
Fraction distilled off up to 206°C under a vacuum of 666 N/m²=2% (distillation ASTM D1160).

The rheological properties were as follows:

kinematic viscosity at 100°C= $4,25 \cdot 10^{-6}$ m²/s

kinematic viscosity at 40°C= $20,30 \cdot 10^{-6}$ m²/s
Viscosity Index (V.I)=114
Pour point=42°C.

The above reported data show that the oil in question, without having undergone any rectification operations, fully satisfies the standards for its use as a high-viscosity-index lubricant base for synthetic lubricants.

Example 2
The procedure of Example 1 is repeated, by feeding the same reactor with 0,30 g of 98%-$H_3PO_4$ and 100 ml of the same mixture of non-terminal olefins. BF₃ is then fed to the autoclave under a pressure of about 3 bar, whereupon a temperature rise of 8°C is noted.

The reaction proceeds for 5 hours, during which the BF₃ pressure drops to about 0,6 bar. The test runs as in Example 1 are repeated and the results are as follows:

|  | % conversion | % selectivity |
|---|---|---|
| 1st run | 82 | 76 |
| 2nd run | 80 | 74 |
| 3rd run | 68 | 73 |
| 4th run | 58 | 72 |
| 5th run | 24 | 72 |

The results show a progressive reduction of the catalytic activity with obvious signs of catalyst poisoning, and, on completion of the reaction, the formation of a black sludge is noted on the reactor bottom.

Example 3
50g of active carbon(cylinders of 4 mm dia.) having a specific surface area of 820 m²/g and a pore volume of 1 ml/g are oven-dried at 120°C for 4 hours. 40 g of an 85%-$H_3PO_4$ solution are separately diluted with 25 ml of water and added to the stirred carbon sample. The procedure is the same as in example 1, and one obtains a product which contains 50 g of $P_2O_5$ per 100 g of carbon. 2,4 g of catalyst are taken and the procedure of example 1 is repeated, using 100 ml of the following mixture of non-terminal C13—C15 olefins:

C13=26,9%; C14=44,3%; C15=28,8%.

Two consecutive runs are carried out using BF₃ under a pressure of about 3 bar, the following results being obtained:

|         | % conversion | % selectivity |
|---------|--------------|---------------|
| 1st run | 81           | 85            |
| 2nd run | 82           | 84            |

The reaction products are treated as described in example 1, and, upon hydrogenation, given an oil having the following characteristics:

kinematic viscosity at 100°C= $4{,}30 \cdot 10^{-6}$ m²/s
kinematic viscosity at 40°C=$21{,}42 \cdot 10^{-6}$ m²/s

Viscosity Index=107
Flammability point=203°C.
pour point=45°C

These properties fulfil the standards for synthethic lubricant bases.

Example 4
The catalyst described in example 3 is heated at 300°C for 14 hours in air, then cooled in a dry nitrogen environment and is used in oligomerization reactions in the same manner as described in said previous example. 1,2 g of catalyst are taken and are used to treat the same amount of non-terminal olefins as described in example 3. Two consecutive runs are carried out, using $BF_3$ under a pressure of about 3 bars, the following results being obtained.

|         | % conversion | % selectivity |
|---------|--------------|---------------|
| 1st run | 84           | 83            |
| 2nd run | 81           | 82            |

The results show that the activity of the catalyst system, when supported on active carbon, is the higher, the higher is the temperature at which the catalyst system concerned is dried.

Example 5
1,2 g of the catalyst prepared as in example 4 is used, following the same procedure as in that example, and using as the monomer a fraction consisting of pure C13 non-terminal olefins. By operating at a temperature of 85°C, in the presence of $BF_3$ and under a pressure of about 3 bar, the conversion of the C13 olefins into oligomers is as high as 78%.
GC-analysis combined with mass-spectrometry shows the presence of the several oligomers and monomers, the respective percentages being:

| monomers  | 22%   |
|-----------|-------|
| dimers    | 63%   |
| trimers   | 13,5% |
| tetramers | 1,5%  |

These analytical data show both the high selectivity of the catalytic system and the absence of any side reactions.

Example 6
50g of silica extrudates having an average dia. of 3 mm, a specific surface area of 330 m²/g and a pore volume of 1 ml/g are oven-dried at 120°C for 4 hours and then treated with a solution composed of 68 g of 85%-$H_3PO_4$ and 8 ml of water, the resultant solution being slowly added to the stirred silica pieces. The sample is oven- dried at 120°C for 14 hours. The acid content, calculated on a $P_2O_5$ basis, on the substrate, is 84 g per 100 g of silica.
1,8 g of the catalyst so prepared are taken and used, as described in example 1, for oligomerizing 100 ml of the C13—C16 olefin mixture described in example 1, in the presence of $BF_3$ under a pressure of about 3 bar for 5 hours.
Oligomerization is repeated as already described and the products obtained in two consecutive runs are GC-analyzed, the results being the following:

|         | % conversion | % selectivity |
|---------|--------------|---------------|
| 1st run | 72           | 76            |
| 2nd run | 70           | 75            |

Example 7
5 g of alumina pellets (dia. 4 mm), having a specific surface area of 220 m²/g and a pore volume of 0,7 ml/g are oven-dried at 120°C for 4 hours and then treated with 41,5 g of 85%-$H_3PO_4$, to which had been added 8 ml of water. The acid has been absorbed by the substrate completely and the product was oven-dried at 120°C for 14 hours in air. The acid content on the substrate, in terms of $P_2O_5$ was 60 g per 100 g of alumina. 2,4 g of the catalyst so prepared are taken and used to oligomerize the same amount of the same C13— C16 olefin fraction as described in example 1, in the presence of $BF_3$ under a pressure of about 5 bar and for 5 hours. Two oligomerization runs are carried out, to give the following results:

|         | % conversion | % selectivity |
|---------|--------------|---------------|
| 1st run | 64           | 78            |
| 2nd run | 62           | 79            |

On completion of the reaction, the solid catalyst has been found unaffected and the $H_3PO_4$ left on the reactor bottom has been acidimetrically titrated at less than 2% of the total quantity present in the system.

**Claims**

1. A process for the oligomerization of non-terminal C10—C20 olefins in the presence of phosphoric acid and boron trifluoride, characterized in that the catalyst used is a substrate of silica, alumina or active carbon having a specific surface area over 200 $m^2$/g and an overall pore volume over 0,5 ml/g impregnated with phosphoric acid ($H_3PO_4$) in a quantity, expressed as $P_2O_5$ of from 20 to 100 parts by weight per 100 parts by weight of the substrate, the reaction taking place in the liquid phase in the presence of gaseous $BF_3$ under a pressure of from 0,3 to 10 bar at a temperature of from 10°C to 130°C, optionally in the presence of a solvent.

2. Process according to claim 1, characterized in that said solvent is a saturated linear or a branched aliphatic hydrocarbon.

## Patentansprüche

1. Verfahren zur Oligomerisation von nichtterminalen C10—C20-Olefinen in Gegenwart von Phosphorsäre und Bortrifluorid, dadurch gekennzeichnet, daß der verwendete Katalysator ein Substrat aus Siliziumdioxid, Aluminiumoxid oder Aktivkohle mit einer spezifischen Oberfläche von über 200 $m^2$/g und einem Gesamtporenvolumen von über 0,5 ml/g ist, der mit Phosphorsäure ($H_3PO_4$) in einer als $P_2O_5$ ausgedrückten Menge von 20 bis 100 Gewichtsteilen je 100 Gewichtsteile des Substrates imprägniert ist, wobei die Reaktion in flüssiger Phase in Gegenwart von gasförmigem $BF_3$ unter einem Druck von 0,3 bis 10 Bar bei einer Temperatur von 10°C bis 130°C, gegebenenfalls in Gegenwart eines Lösungsmittels, ausgeführt wird.

2. Verfahren nach Anspruch 1, dadurch gekennzeichnet, daß das genannte Lösungsmittel ein gesättigter linearer oder ein verzweigter aliphatischer Kohlenwasserstoff ist.

## Revendications

1. Procédé pour l'oligomérisation d'oléfines non terminales en $C_{10}$—$C_{20}$ en présence d'acide phosphorique et de trifluorure de bore, caractérisé par le fait que le catalyseur utilisé est un support de silice, d'alumine ou charbon actif ayant une surface spécifique supérieure à 200 $m^2$/g et un volume total des pores supérieur à 0,5 ml/g, imprégné avec de l'acide phosphorique ($H_3PO_4$) en une quantité, exprimée en $P_2O_5$, de 20 à 100 parties en poids, pour 100 parties en poids du support, la réaction ayant lieu dans la phase liquide en présence de $BF_3$ gazeux, sous une pression de 0,3 à 10 bars, à une température de 10°C à 130°C, éventuellement en présence d'un solvant.

2. Procédé selon la revendication 1, caractérisé par le fait que ledit solvant est un hydrocarbure aliphatique saturé à chaîne linéaire ou à chaîne ramifiée.